# FASCICULE DE BREVET EUROPEEN

(11) **EP 2 688 396 B1**
(45) Date de publication et mention de la délivrance du brevet: **20.01.2016**
(21) Numéro de dépôt: 12714814.6
(22) Date de dépôt: 19.03.2012
(51) Int. Cl.: A01K 67/02, A01K 67/027

(54) **PROCEDE D'OBTENTION D'UN HYBRIDE INTERSPECIFIQUE TRIPLOÏDE DE MAIGRE ET D'UNE OMBRINE**
VERFAHREN ZUR HERSTELLUNG EINES TRIPLOIDEN INTERSPEZIFISCHEN HYBRIDS DER SPEZIES ADLERFISCH UND ROTER TROMMLER
METHOD FOR OBTAINING A TRIPLOID INTERSPECIFIC HYBRID OF MEAGRE AND RED DRUM

(30) Priorité: 23.03.2011 FR 1152396
(43) Date de publication de la demande: 29.01.2014
(73) Titulaire: Scea Les Poissons Du Soleil, 34540 Balaruc Les Bains (FR)
(72) Inventeur: BALMA, Georges, F-34200 Sete (FR)
(74) Mandataire: Rhein, Alain
(86) Numéro de dépôt international: PCT/FR2012/050579
(87) Numéro de publication internationale: WO 2012/131223

(56) Documents cités:
- WO-A1-2006/067356
- ALICIA FELIP: "STERILIZING FISH TO PROTECT BIODIVERSITY AND IMPROVE QUALITY", THE FUTURE PROSPECTS FOR AQUACULTURE BREEDING IN EUROPE, 8 octobre 2008 (2008-10-08), pages 1-19, XP055008775,
- LORENZO COLOMBO ET AL: "Towards an integration between chromosome set manipulation, intergeneric hybridization and gene transfer in marine fish culture", CAHIERS OPTIONS MEDITERRANEENNES, vol. 34, 1 janvier 1998 (1998-01-01), pages 77-122, XP055008654, ISSN: 1022-1379
- PIFERRER F ET AL: "Polyploid fish and shellfish: Production, biology and applications to aquaculture for performance improvement and genetic containment", AQUACULTURE, ELSEVIER, AMSTERDAM, NL, vol. 293, no. 3-4, 16 août 2009 (2009-08-16), pages 125-156, XP026218217, ISSN: 0044-8486, DOI: 10.1016/J.AQUACULTURE.2009.04.036 [extrait le 2009-05-03]
- ANNE HENDERSON-ARZAPALO ET AL: "A Comparison of Black Drum, Red Drum, and their Hybrid in Saltwater Pond Culture", JOURNAL OF THE WORLD AQUACULTURE SOCIETY, vol. 25, no. 2, 1 juin 1994 (1994-06-01), pages 289-296, XP055008777, ISSN: 0893-8849, DOI: 10.1111/j.1749-7345.1994.tb00193.x

## Description

L'invention concerne l'obtention d'un poisson hybride interspécifique triploïde de maigre et d'un autre poisson appartenant à la famille des Sciaenidés.

La présente invention entre dans le domaine de l'aquaculture, et plus particulièrement de la pisciculture.

L'apport quantitatif de la pêche mondiale aux marchés de produits aquatiques, notamment pour couvrir la consommation humaine en poissons, est stable depuis une vingtaine d'années. Mais surtout, l'état actuel des stocks de poissons sauvages ne permet généralement pas, pour cause de surpêche malheureusement de plus en plus fréquente, de prévoir une modification de cette tendance, particulièrement dans un contexte d'augmentation de la population mondiale.

De fait, l'approvisionnement futur du marché mondial repose donc sur l'essor de l'aquaculture et en particulier sur l'essor de la pisciculture, qui désigne plus précisément l'élevage des poissons. Si aujourd'hui les produits aquatiques d'élevage ont significativement augmenté leur part de marché en vingt ans, l'amélioration des espèces proposées à la pisciculture et leur diversification reste une nécessité fondamentale.

En Atlantique et en Méditerranée, la pisciculture marine concerne une large variété d'espèces de poisson différentes, et notamment le saumon atlantique (*Salmo salar*), le bar (*Dicentrarchus labrax*), la daurade royale (*Sparus aurata*) mais également le thon rouge (*Thunnus thynnus*), le flétan (*Hippoglossus hippoglossus*), les soles (*Solea solea et Solea senegalensis),* la morue (*Gadus morua*) ou encore le maigre (*Argyrosomus regius*).

Le maigre, ou *Argyrosomus regius,* appartient à la famille des Sciaenidés. Il s'agit d'un poisson marin d'Europe rare à la pêche. Les prises annuelles sont variables et, si elles peuvent atteindre 10 000 tonnes, cela reste une contribution mineure aux volumes de pêche européens. De plus, la taille des poissons pêchés est très inégale. Ces caractéristiques induisent une hausse des prix qui reflète l'intérêt des consommateurs pour le maigre.

En effet, le maigre possède une chair fine et savoureuse très appréciée et dont la qualité est comparable à celle d'autres espèces marines élevées. Le taux bas de lipides et leur équilibre dans le muscle confère à l'espèce une haute valeur diététique. Cette espèce possède donc d'indéniables qualités gustatives et nutritionnelles.

Sa conquête du marché est donc potentiellement freinée par sa faible disponibilité. D'autant que le maigre présente les caractères d'un bon candidat à l'aquaculture car, outre la qualité de sa chair, il présente une croissance rapide sous les latitudes européennes, conduisant à une taille commerciale dans un délai raisonnable, cela même si l'information sur la biologie de l'espèce en captivité est réduite du fait de sa faible expansion. Cependant, la production aquacole de maigre est récente et limitée à la côte méditerranéenne française, à la Corse et à l'Italie. Cette production a atteint à peine 1000 tonnes par an en 2004.

Enfin, la morphologie du maigre rend le traitement de sa carcasse, en vue de sa transformation par exemple en produits surgelés tout prêts, sujet à de nombreux déchets.

Les inventeurs sont parvenus à maîtriser la reproduction en captivité du maigre. Cependant, des solutions restaient à trouver pour améliorer l'espèce en vue de maximiser son potentiel aquicole.

Dans le domaine de la pisciculture, une technique connue pour obtenir des animaux de taille importante est le blocage de la maturation sexuelle. Cette technique permet d'une part d'éviter que les aliments ingérés par les poissons ne soient détournés vers la fonction de reproduction ; ceux-ci sont alors exclusivement utilisés pour la croissance de l'animal. D'autre part, la maturation sexuelle est responsable de modifications physiologiques chez l'animal qui vont induire une diminution de la qualité du produit.

En ce qui concerne l'aquaculture de la morue (*Gadus morua*), le blocage de la maturation s'effectue par un maintien des animaux à la lumière artificielle. Cependant, cette technique s'est avérée peu concluante.

Une autre technique connue consiste en la réalisation d'une hybridation interspécifique. Cette technique présente des avantages par rapport à l'hybridation intraspécifique qui peut entrainer des problèmes de perte de diversité génétique. L'hybridation interspécifique est utilisée pour la production d'animaux stériles ce qui évite une reproduction non contrôlée entre les souches domestiques et les stocks sauvages. Cela permet de protéger les milieux naturels d'une éventuelle pollution génique ou environnementale liée à l'élevage de souches domestiques ou d'espèces non indigènes. De plus, l'hybridation interspécifique n'entraîne pas de manipulation génétique du produit. En effet, la commercialisation d'un organisme génétiquement modifié nécessiterait l'obtention d'une autorisation administrative préalable et pourrait déranger le consommateur.

On connait par exemple, dans l'état de la technique, notamment du document WO 2006/067356, un procédé consistant à croiser un bar *Dicentrarchus labrax* avec un bar *Dicentrarchus punctatus* puis de réaliser un traitement d'induction de la triploïdie sur la descendance obtenue.

Il existe également des procédés de triploïdisation de bar (*Dicentrarchus labrax*) qui permettent l'obtention d'environ 100% d'individus triploïdes, notamment par croisement entre individus tétraploïdes et diploïdes. Ces techniques permettent l'amélioration de certains caractères de qualité, comme le rendement de carcasse. Cependant, les performances de croissance des triploïdes de bar sont généralement inférieures de 20% à celle des témoins diploïdes.

De plus, aucun procédé ne permet actuellement une amélioration des qualités du maigre.

L'invention offre la possibilité de proposer une solution à la nécessité d'améliorer les qualités intrinsèques du maigre en vue de sa production aquicole tout en palliant les divers inconvénients de l'état de la technique.

A cet effet, l'invention concerne un procédé d'obtention d'un hybride interspécifique triploïde de maigre et d'une ombrine appartenant à la famille des Sciaenidés comprenant la réalisation d'un traitement d'induction de la triploïdie sur la descendance obtenue par un maigre appartenant au genre Argyrosomus, et, une ombrine Sciaenops ocellatus.

Ce procédé permet d'obtenir des hybrides stériles. Par ailleurs, il ne constitue pas un procédé essentiellement biologique. En effet, le traitement d'induction de la triploïdie est une étape technique essentielle dudit procédé, nécessitant une intervention humaine, et transposable à l'échelle industrielle.

Avantageusement, le traitement d'induction de la triploïdie consiste à soumettre les oeufs fécondés à un choc hyperbare ou à un choc thermique.

Préférentiellement, le choc hyberbare est réalisé à une pression de 400 à 800 bars pendant 1 à 4 minutes, et le choc thermique est réalisé à une température de -5°C à +5°C pendant 10 à 30 minutes.

Selon une caractéristique avantageuse, le traitement d'induction de la triploïdie est réalisé 2 à 10 minutes après fécondation des oeufs.

Avantageusement, le procédé selon l'invention est réalisé sur la descendance obtenue par le croisement d'un maigre *Argyrosomus regius* mâle avec une ombrine *Sciaenops ocellatus* femelle ou d'un maigre *Argyrosomus regius* femelle avec une ombrine *Sciaenops ocellatus* mâle.

L'invention concerne encore un hybride triploïde de maigre et d'ombrine obtenu par le procédé selon l'invention.

Les avantages qui découlent de la présente invention consistent, essentiellement, en l'obtention d'un hybride stérile, présentant un bon rendement en élevage et des caractéristiques morphologiques avantageuses. En outre, le profil adaptatif de l'hybride à des conditions de températures plus variées que les espèces dont il est issu est tout à fait intéressant, et offre une vraie valeur ajoutée pour les conditions d'élevage en Europe.

D'autres buts et avantages de la présente invention apparaîtront à la description qui va suivre, qui sera complétée par les figures suivantes :
- La figure 1 représente un graphique illustrant le régime alimentaire d'hybrides à l'état larvaire pendant les 30 premiers jours de leur vie, selon les modalités de l'exemple 2 ci-après.
- La figure 2 représente un graphique illustrant la température d'élevage appliquée aux larves hybrides de l'exemple 2, pendant les 29 premiers jours de leur vie.
- La figure 3 représente un graphique illustrant la température d'élevage appliquée aux larves hybrides de l'exemple 2, du 30^{ème} au 50^{ème} jour d'élevage.
- La figure 4 représente un graphique illustrant la température des bassins pendant la période de pré-grossissement et de croissance de l'exemple 3.
- La figure 5 représente un graphique illustrant la courbe de croissance du 50^{ème} au 114^{ème} jour pour les groupes d'alevins hybrides de l'exemple 3, avec des maigres témoins.
- La figure 6 représente des hybrides obtenus par le procédé décrit, au 29^{ème} jour de vie à gauche et au 64^{ème} jour de vie à droite.
- La figure 7 représente un hybride obtenu par le procédé décrit, au 114^{ème} jour de vie.

La présente invention concerne le domaine de l'aquaculture, et plus particulièrement de la pisciculture. L'invention a plus particulièrement trait à l'obtention d'un poisson hybride interspécifique triploïde de maigre et d'un autre poisson appartenant à la famille des Sciaenidés, l'ombrine tropicale (*Sciaenops ocellatus*).

La famille des Scianidés, à laquelle appartiennent le maigre et l'ombrine, en particulier l'ombrine tropicale, est une grande famille de poissons regroupant plus de 200 espèces réparties sous environ 70 genres.

Le genre Argyrosomus comprend notamment l'espèce *Argyrosomus regius,* communément appelée le « maigre ». Le maigre est un poisson marin de l'Atlantique et de la Méditerranée. Il a un corps fusiforme, légèrement aplati sur les flancs. Son profil de dos est nettement convexe, et sa ligne ventrale pratiquement rectiligne. La coloration du dos est grise, les flancs et la nageoire dorsale sont gris argenté et brillants. Une ligne latérale présente une bande noire pointillée qui va jusqu'à la nageoire caudale. Les yeux sont petits, le museau présente une grande bouche. Il possède des canines très visibles. De façon caractéristique, la nageoire dorsale du maigre se présentant en deux parties, la nageoire dorsale arrière est nettement plus longue que sa nageoire dorsale antérieure. Sa taille moyenne la plus courante est de 50 centimètres à 1 mètre, mais il peut atteindre 2 mètres pour 100 kilogrammes en milieu naturel. Il se pêche en zone côtière par moins de 80 mètres, sur des fonds sableux ou vaseux. Le maigre marquant une préférence pour la couche d'eau la plus proche du fond, son mode de vie est qualifié de semi-pélagique à tendance démersale. Un comportement grégaire est observé pendant la phase juvénile ainsi que lors des migrations de reproduction. En dehors de cette période, les adultes paraissent vivre isolés ou en petits groupes. La maturation sexuelle est atteinte entre 3 et 5 ans (à 4-5 kg). La reproduction a lieu d'octobre à mai au maximum. Le maigre ne supporte pas des températures inférieures à 10°C. En aquaculture, le maigre est élevé entre environ 19°C et 20°C.

Le genre Sciaenops comprend notamment l'espèce *Sciaenops ocellatus,* communément appelée « ombrine » et plus particulièrement « ombrine tropicale », ou encore « red drum ». L'ombrine tropicale habite principalement les eaux côtières et estuariennes du nord-est de l'Océan Atantique jusqu'au Golfe du Mexique. Son habitat étant, en milieu naturel, totalement distinct de celui du maigre, les deux espèces ne sont pas susceptibles de se rencontrer. L'ombrine tropicale a une couleur orange-rouge caractéristique qui peut varier du gris au bronze rouge et a la particularité d'avoir un ou plusieurs points noirs près de la base du pédoncule caudal appelé ocelle. L'ombrine tropicale peut atteindre une taille allant jusqu'à plus d'1 mètre, voire 1,50 mètres et peut peser environ 40 kg. L'ombrine tropicale est une espèce eurytherme et euryhaline, carnivore. Les larves se nourrissent essentiellement d'invertébrés tandis que les adultes préfèrent les poissons, les crevettes et les crabes. Les juvéniles sont trouvés dans les baies et les estuaires jusqu'à leur maturité sexuelle, les poissons vivant le plus au nord se déplacent en hiver vers le sud pour trouver des eaux plus chaudes. La maturation sexuelle est atteinte entre 3 à 5 ans (à 4-5 kg). La reproduction a lieu d'août à octobre, soit en décalage complet avec la période de reproduction du maigre. L'ombrine tropicale ne supporte pas des températures inférieures à 5°C. En aquaculture, l'ombrine tropicale est élevée entre environ 27°C et 28°C. L'ombrine possède donc un profil thermique plus large que celui du maigre, car bien qu'adaptée à des eaux chaudes, elle supporte des conditions encore plus froides que le maigre.

Le croisement entre deux membres d'une même famille phylogénétique, en particulier si ces deux membres possèdent des périodes de reproduction ne se recouvrant pas, nécessite la mise en oeuvre des étapes générales suivantes :
- Collecte du sperme d'une des deux espèces et conservation en frais ou en congelé,
- Contrôle de l'état de maturation des gonades des femelles de l'autre espèce,
- Collecte des oeufs des femelles, par exemple par pression abdominale,
- Si nécessaire, stabilisation et contrôle de l'hygrométrie et de la température des ovocytes récupérés,
- Décongélation du sperme de l'espèce mâle (si congélation) et contrôle de sa qualité et de sa mobilité,
- Fécondation artificielle des ovocytes avec le sperme,
- Incubation des oeufs obtenus, avec un contrôle régulier de l'incubation et de la division cellulaire,
- Contrôle de l'éclosion,
- Élevage larvaire,
- Sevrage,
- Pré-grossissement, débouchant sur la phase de croissance.

L'hybride obtenu ne ressemble ni au maigre ni à l'ombrine. Au contraire, ledit hybride possède certaines caractéristiques morphologiques du maigre et d'autres de l'ombrine. L'hybride triploïde présente notamment une forme du corps plus proche de celle du maigre et une couleur ressemblant plus à celle de l'ombrine.

Les exemples qui vont suivre retracent un mode détaillé de réalisation de l'invention.

### Exemple 1 : Réalisation d'un croisement entre Argyrosomus regius et Sciaenops ocellatus

Les deux espèces ne se reproduisant pas au même moment, l'hybridation se fait par reproduction artificielle avec congélation du sperme des mâles d'une espèce en attendant que les femelles de la deuxième espèce entrent en reproduction. Le croisement peut se faire dans les deux sens, les mâles d'une espèce étant hybridés avec les femelles de la seconde espèce et vice-versa.

De façon classique, les femelles ombrines ont été induites par injection d'hormone. A cette fin, les femelles présentant un stade de maturation ovocytaire adéquat sont sélectionnées, et on leur injecte de la LHRHa (hormone de libération de la lutéinostimuline) à la dose de 10mg/kg de poids vif, puis on les isole en structure de ponte. Soixante-dix heures après l'induction des femelles, l'on récupère les gamètes mâles et femelles, et l'on procède alors à la fécondation artificielle en mélangeant tout d'abord les ovocytes et le sperme avant de rajouter l'eau de mer dans les proportions respectives de 1000:1:500.

C'est à ce moment que l'on réalise l'induction de la triploïdie des oeufs par choc hyperbare ou thermique.

Le choc hyperbare est préférentiellement réalisé à une pression allant de 400 à 800 bars pendant 1 à 4 minutes.

Alternativement, les oeufs fécondés peuvent être soumis à un choc thermique, à une température allant de -5°C à 5°C pendant 10 à 30 minutes.

De manière générale, le traitement visant l'induction de la triploïdie est réalisé 2 à 10 minutes après la fécondation des oeufs. Les hybrides issus de ce procédé sont hybrides triploïdes à approximativement 100 %.

Néanmoins, pour vérifier la triploïdie et/ou évaluer les performances d'induction de la triploïdie, l'on peut procéder à l'analyse des cellules de l'hybride à tester, soit en cytométrie de flux à iodure de propidium, soit par analyse visuelle de leur caryotype. La vérification par cytométrie de flux peut s'effectuer avec un cytomètre de flux de type Facscan (Becton Dickinson). Le contenu ADN de chaque poisson est alors mesuré et comparé aux cellules de l'échantillon témoin (diploïde 2n), issu par exemple d'un stock témoin non soumis au traitement d'induction de la triploïdie préalablement décrit. Si l'on procède par caryotypage, il suffit de compter le nombre de chromosomes que possède l'hybride testé, les triploïdes ayant 3n chromosomes, soit 72 chromosomes, les diploïdes n'en ayant que 2n, soit 48 chromosomes. Ces techniques de mesure par cytométrie de flux et/ou de caryotypage sont conventionnelles pour l'homme du métier.

Les hybrides triploïdes obtenus présentent une morphologie générale proche de celle du maigre associée à une couleur des écailles comportant des reflets jaunes caractéristiques de l'ombrine.

L'homme du métier pourra ainsi reconnaître et sélectionner, par un simple contrôle visuel, les hybrides obtenus et discerner les hybrides triploïdes des hybrides qui auraient pu ne pas avoir été rendus triploïdes.

L'homme du métier pourra également considérer l'utilisation de marqueurs moléculaires présents au niveau de l'ADN pour identifier les hybrides obtenus par le procédé selon l'invention.

Les oeufs fécondés induits sont alors incubés. Après éclosion, l'on procède à l'élevage larvaire.

### Exemple 2 : Elevage larvaire de larves hybrides obtenues par croisement de femelles ombrines avec des mâles maigres

Les larves utilisées proviennent d'oeufs fécondés en Martinique. Les larves ont été reçues dans 4 cubitainers de 30 litres à l'âge d'un jour, à Palavas en France, où l'élevage larvaire a été effectué. Elles ont été mises en élevage dans quatre bassins cylindro-coniques de 500 litres en circuit fermé (le bac 1 contenant des ombrines témoins et les bacs 2, 3, 4 des hybrides). Elles n'ont pas été dénombrées de manière à ne pas ajouter une manipulation dangereuse juste après le stress important du voyage. Les conditions d'élevage sont détaillées ci-après.

### Conditions de lumière

Du jour 1 (J1) au jour 35 (J35), les animaux sont soumis à un éclairage constant d'intensité variable.

De J1 à J10, l'intensité lumineuse générée par des tubes néon blanc industrie est de 300 lux à la surface de l'eau. De plus, un apport d'algues journalier limite la pénétration de lumière dans le milieu d'élevage tout en le stabilisant (eau verte).

De J11 à J35 l'intensité lumineuse est réduite à 50 lux à l'aide de toile ombrage tandis que les algues sont supprimées.

Du jour 35 (J35) au jour 40 (J40), les larves sont progressivement amenées à une alternance 16 h jour/8h nuit en 3 étapes successives (22h jour/2h nuit ; 19h jour/5h nuit et enfin 16h jour/8 nuit).

### Conditions hydrauliques

Le renouvellement de l'eau est fixé à 3 % au début de l'élevage, à 15 % à 10 jours et augmenté à 25 % à partir de 16 jours. Il est assuré par une entrée d'eau en subsurface tournée vers la paroi pour éviter l'établissement d'un courant dans le bassin. La sortie d'eau est en surface, un léger bullage d'air par le fond du bassin assure un lent mouvement de convection.

Pendant toute cette période des écrémeurs de surface sont en place sur les bassins afin d'éliminer la couche huileuse générée par l'alimentation.

La salinité est naturelle (35-38g/l) et des analyses physico-chimiques de l'eau sont réalisées deux fois par semaine.

### Conditions d'alimentation

Les larves sont nourries uniquement de rotifères dès leur arrivée et jusqu'à l'âge de 6 jours. De 7 jours à 10 jours, elles sont nourries d'un mélange rotifères - nauplii d'Artemia (A1) et restent sur A1 jusqu'à l'age de 15 jours. Dès le 16éme jour une petite quantité de micro-particules (Gemma 0.1) est ajoutée à l'alimentation puis augmentée progressivement jusqu'au sevrage total à l'âge de 31 jours selon la séquence.

| Date | Aliment | Proportion (%) |
|---|---|---|
| J16-J18 | Gemma 0.1 | 100 |
| J19-J22 | Gemma 0.1 / Gemma 0.2 | 60/40 |
| J23-J26 | Gemma 0.2 | 100 |
| J27-J31 | Gemma 0.2 / Gemma 0.3 | 50/50 |

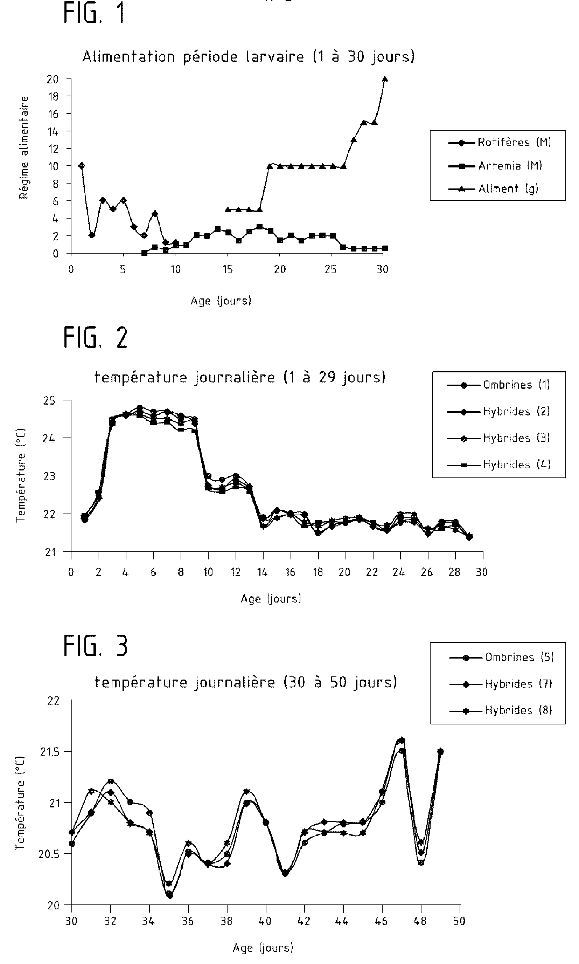

La séquence alimentaire est présentée dans la Figure 1 où l'unité des courbes « Rotifères » et « Artemia » est le million d'individus de proies vivantes par jour et l'unité d'aliment inerte, représenté par la courbe « Aliment » est le gramme/jour.

### Conditions de température

A la suite du voyage à température fraîche (22°C), les larves sont rapidement passées de 22° C à 24° C et sont maintenues aux alentours de 24° C jusqu'à l'âge de 9 jours. Cette température a été choisie entre les températures d'élevage des espèces d'origine (l'ombrine étant élevée entre environ 27 et 28°C et le maigre entre environ 19 et 20°C).

De 10 jours à 50 jours, la température est ajustée entre 21 et 22° C afin de tester les performances de l'hybride en conditions métropolitaines. Le graphique en Figure 2 indique les variations quotidiennes observées au cours de l'élevage sur les 4 bassins de 500 1 sur les 29 premiers jours. A 30 jours, à la suite d'une période de cannibalisme, les hybrides ont été triés, dénombrés et séparés en deux lots. Le lot des plus gros est alors constitué de 318 individus et le lot des plus petits de 203 individus. Le bassin des ombrines ne comporte que 7 individus. Les températures relevées quotidiennement entre le 30^{ème} et le 50^{ème} jour d'élevage dans les 3 bassins sont représentées dans le graphique de la Figure 3.

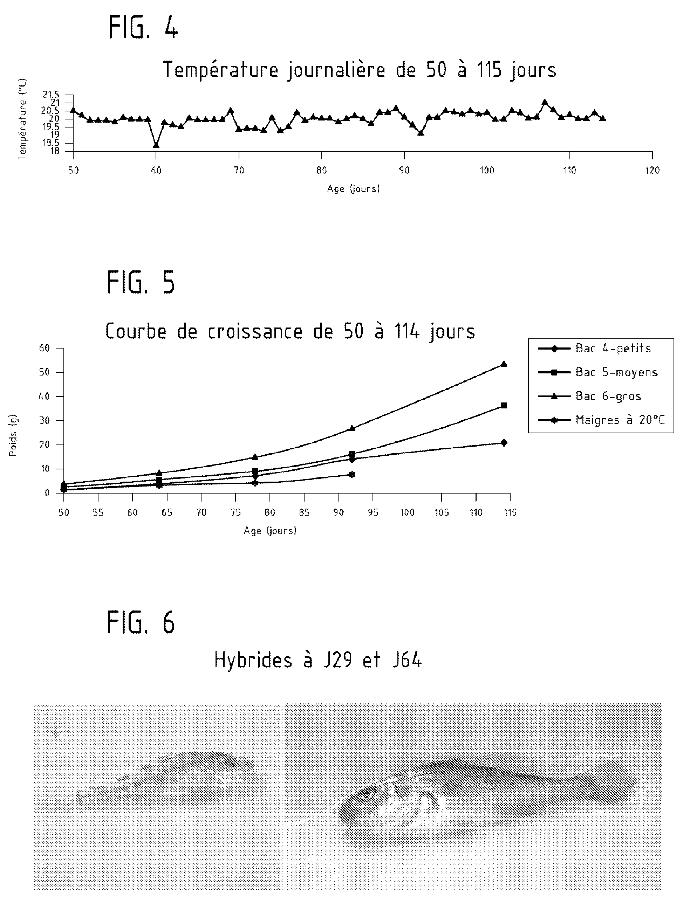

La figure 6 à gauche représente des hybrides obtenus par le procédé décrit, au 29^{ème} jour de vie.

Les hybrides ont surprenamment bien répondu aux conditions d'élevage. Plus particulièrement la mortalité a été réduite à seulement quelques individus sur la durée du processus de sevrage (une dizaine sur une population évaluée à moins de 1000 individus).

### Exemple 3 : Pré-grossissement et croissance des alevins issus de l'élevage larvaire de l'exemple 2

A 50 jours, les alevins issus de l'élevage larvaire décrit dans l'exemple 2 sont transférés dans une salle de pré-grossissement équipée de bassins carrés de 450 litres à fond plat, qui fonctionnent en circuit fermé. L'intensité lumineuse est de 50 lux et la température est en moyenne de 20° C avec les variations présentées dans le graphe de la Figure 4, qui indique les températures relevées quotidiennement entre le 50^{ème} et le 114^{ème} jour d'élevage

Trois lots d'hybrides sont réalisés :
- lot des « petits » : 112 individus de poids moyen 1.52 g
- lot des « moyens » : 199 individus de poids moyen 2.49 g
- lot des « gros » : 199 individus de poids moyen 3.94 g.

Les animaux sont alimentés au départ avec un mélange Gemma micro 0.3 / Al1 (Le Gouessant) à l'aide de tapis distributeurs puis sont rapidement habitués au self feeders uniquement sur aliment le Gouessant (Al1 ; A12). A J50, l'apparition de lésions sur les nageoires caudales et dorsales pouvant être due à des carences d'alimentation, les animaux ont été nourris à l'aide d'aliment Skretting 1.9 qui est généralement utilisé chez le maigre. Le taux de rationnement est de l'ordre de 10% par jour. Le changement de granulé semble avoir eu un effet favorable sur l'état des poissons.

Ces conditions d'élevage ont permis d'obtenir les courbes de croissances représentées en Figure 5. Ces courbes ont été établies à partir de biométries exhaustives ou par échantillonnage de 30 individus à un rythme bimensuel.

La figure 6 à droite représente des hybrides obtenus par le procédé décrit, au 64^{ème} jour de vie.

Nous avons constaté que les hybrides, petits, moyens ou gros, présentent avantageusement une croissance plus importante que le maigre, en conditions métropolitaines, au moins jusqu'au 92^{ème} jour.

À partir de l'âge de 114 jours, les poissons sont placés dans des bassins de 5 m³, en circuit fermé et à une température de 20° C. Le cheptel restant est alors de 499 individus.

Les hybrides obtenus possèdent une chair fine à fort potentiel commercial, une croissance rapide et présentent l'avantage de pouvoir s'adapter à des profils thermiques plus divers que le maigre. Ceci constitue un avantage indéniable pour le développement commercial ultérieur des hybrides selon l'invention, ces derniers pouvant ainsi être cultivés dans des conditions thermiques très diverses.

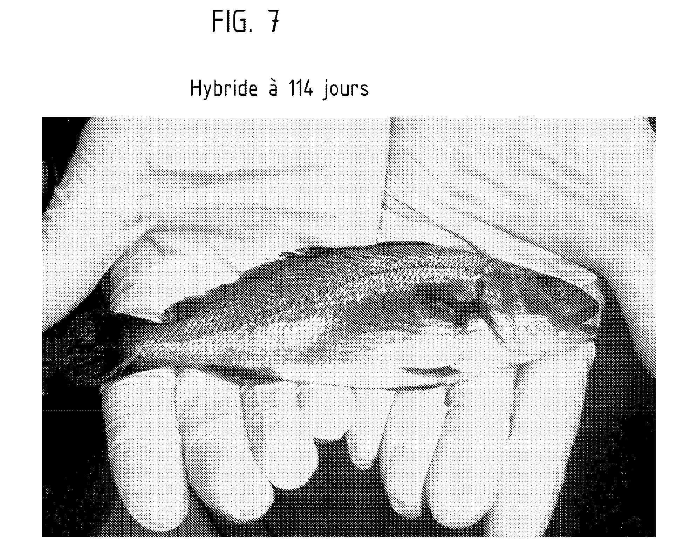

La morphologie de l'hybride obtenu, qui possède une tête réduite par rapport au maigre, comme visible en figure 7, permet d'atteindre des gains importants sur les rendements de transformation, ce qui représente un avantage très intéressant par rapport à l'objectif initial qui était l'obtention d'un hybride à fort potentiel commercial.

Tel qu'il ressort de la description qui précède, la présente invention vient répondre de façon très pertinente au problème posé. En particulier, elle permet la diversification du cheptel disponible à la pisciculture, avec un hybride à plus forte valeur marchande. L'on rappellera que les produits aquatiques sont amenés à jouer un rôle important dans les pays en voie de développement, où la consommation mondiale de protéines animales a plus que doublé au cours des 30 dernières années.

En effet, l'élevage de nouvelles « espèces » en pisciculture est justifié par une amélioration de l'adaptation aux conditions variées de l'environnement observées en Europe et dans le Monde, grâce à la disponibilité d'un éventail d'espèces variées. C'est aussi grâce à ces nouvelles « espèces » que l'on peut espérer une augmentation des performances observées en élevage. Par ailleurs, la diversification du cheptel répond à une meilleure adéquation aux demandes des distributeurs et des consommateurs, tout en permettant, comme c'est le cas avec l'hybride obtenu qui permet une transformation plus aisée et plus rapide, une meilleure productivité.

## Revendications

1. Procédé d'obtention d'un hybride interspécifique triploïde de maigre et d'une ombrine appartenant à la famille des Sciaenidés dans lequel on réalise un traitement d'induction de la triploïdie sur la descendance obtenue par un maigre appartenant au genre Argyrosomus, et, une ombrine Sciaenops ocellatus.

2. Procédé selon la revendication 1 caractérisé en ce la descendance obtenue consiste en des oeufs fécondés artificiellement, lesdits oeufs étant obtenus en mélangeant le sperme d'un maigre mâle avec les ovocytes d'une ombrine Sciaenops ocellatus femelle, ou, en mélangeant les ovocytes d'un maigre femelle avec le sperme d'une ombrine Sciaenops ocellatus mâle.

3. Procédé selon la revendication 2, **caractérisé en ce que** le traitement d'induction de la triploïdie consiste à soumettre les oeufs fécondés à un choc hyperbare.

4. Procédé selon la revendication 3, **caractérisé en ce que** le choc hyperbare est réalisé à une pression de 400 à 800 bars pendant 1 à 4 minutes.

5. Procédé selon la revendication 2, **caractérisé en ce que** le traitement d'induction de la triploïdie consiste à soumettre les oeufs fécondés à un choc thermique.

6. Procédé selon la revendication 5, **caractérisé en ce que** le choc thermique est réalisé à une température de -5°C à +5°C pendant 10 à 30 minutes.

7. Procédé selon l'une quelconque des revendications 1 à 6, **caractérisé en ce que** le traitement d'induction de la triploïdie est réalisé 2 à 10 minutes après fécondation des oeufs.

8. Hybride triploïde de maigre et d'ombrine Sciaenops ocellatus obtenu selon le procédé des revendications 1 à 7.

## Patentansprüche

1. Verfahren zur Gewinnung einer triploiden Arthybride aus einem Adlerfisch und einem zur Familie der Sciaenidae gehörenden Umberfisch, bei dem eine Induktionsbehandlung der Triploidie auf der aus einem zur Art der Argyrosomus gehörenden Adlerfisch und einem Sciaenops ocellatus Umberfisch erhaltenen Nachzucht durchgeführt wird.

2. Verfahren nach Anspruch 1, bei dem die erhaltene Nachzucht aus künstlich befruchteten Eiern besteht, wobei die Eier durch Mischen des Spermas eines männlichen Adlerfisches mit den Oozyten eines weiblichen Sciaenops Ocellatus Umberfisches oder durch Mischen der Oozyten eines weiblichen Adlerfisches mit dem Sperma eines männlichen Sciaenops Ocellatus Umberfisches erhaltenen werden.

3. Verfahren nach Anspruch 2, **dadurch gekennzeichnet, dass** die Induktionsbehandlung der Triploidie darin besteht, die befruchteten Eier einem hyperbaren Schock zu unterwerfen.

4. Verfahren nach Anspruch 3, **dadurch gekennzeichnet, dass** der hyperbare Schock bei einem Druck von 400 bis 800 bar während 1 bis 4 Minuten durchgeführt wird.

5. Verfahren nach Anspruch 2, **dadurch gekennzeichnet, dass** die Induktionsbehandlung der Triploidie darin besteht, die befruchteten Eier einem thermischen Schock zu unterwerfen.

6. Verfahren nach Anspruch 5, **dadurch gekennzeichnet, dass** der thermische Schock bei einer Temperatur von -5°C bis +5°C während 10 bis 30 Minuten durchgeführt wird.

7. Verfahren nach einem der Ansprüche 1 bis 6, **dadurch gekennzeichnet, dass** die Induktionsbehandlung der Triploidie 2 bis 10 Minuten nach Befruchtung der Eier durchgeführt wird.

8. Triploide Hybride aus einem Adlerfisch und einem Sciaenops ocellatus Umberfisch, die mit dem Verfahren nach den Ansprüchen 1 bis 7 erhalten wird.

## Claims

1. A method for obtaining a triploid interspecific hybrid of meagre and a red drum belonging to the family of Sciaenidae, wherein an induction treatment of the triploidy is performed on the progeny obtained by a meagre belonging to the Argyrosomus species and a Sciaenops ocellatus red drum.

2. The method according to claim 1, wherein the progeny obtained consists of artificially fertilized eggs, said eggs being obtained by mixing the sperm of a male meagre with the oocytes of a female Sciaenops ocellatus red drum, or by mixing the oocytes of a female meagre with the sperm from a male Sciaenops ocellatus red drum.

3. The method according to claim 2, wherein the triploidy inducing treatment consists in subjecting the fertilized eggs to a hyperbaric shock.

4. The method according to claim 3, wherein the hyperbaric shock is performed at a pressure between 400 and 800 bars for 1 to 4 minutes.

5. The method according to claim 2, wherein the triploidy inducing treatment consists in subjecting the fertilized eggs to a thermal shock.

6. The method according to claim 5, wherein the thermal shock is performed at a temperature between -5°C and +5°C for 10 to 30 minutes.

7. The method according to any one of claims 1 to 6, wherein the triploidy inducing treatment is performed 2 to 10 minutes after fertilization of the eggs.

8. Hybrid triploid from meagre and Sciaenops ocellatus red drum obtained by the process according to claims 1 to 7.
